# EUROPÄISCHE PATENTSCHRIFT

(11) **EP 4 511 344 B1**
(45) Veröffentlichungstag und Bekanntmachung des Hinweises auf die Patenterteilung: **09.07.2025**
(21) Anmeldenummer: 23730039.7
(22) Anmeldetag: 27.05.2023
(51) Int. Cl.: C04B 35/16, A61C 13/00, A61K 6/802, A61K 6/827, B28B 1/00, B33Y 70/00, C04B 35/19, C04B 35/195, C04B 35/626, C04B 35/632, C04B 35/634, C04B 35/64, B33Y 70/10

(54) **SATZ VON CHEMIKALIEN FÜR DIE HERSTELLUNG EINER KERAMIKDISPERSION ODER EINES KERAMIKGELS**
USE OF CHEMICALS FOR THE PRODUCTION OF A CERAMIC DISPERSION OR A CERAMIC GEL
UTILISATION DE PRODUITS CHIMIQUES POUR LA PRODUCTION D'UNE DISPERSION CÉRAMIQUE OU D'UN GEL CÉRAMIQUE

(30) Priorität: 27.05.2022 DE 102022113425
(43) Veröffentlichungstag der Anmeldung: 26.02.2025
(73) Patentinhaber: CERAMIST ONE GMBH, 65183 Wiesbaden (DE)
(72) Erfinder: MÜLLER, Dirk, 65193 Wiesbaden (DE)
(74) Vertreter: WSL Patentanwälte Partnerschaft mbB
(86) Internationale Anmeldenummer: PCT/EP2023/064303
(87) Internationale Veröffentlichungsnummer: WO 2023/227794

(56) Entgegenhaltungen:
- EP-A1- 3 659 989
- EP-A1- 3 919 092
- EP-A2- 1 243 231
- US-A1- 2017 128 174
- US-A1- 2018 303 723
- US-A1- 2021 238 099
- BAUMGARTNER SONJA ET AL: "Stereolithography-based additive manufacturing of lithium disilicate glass ceramic for dental applications", MATERIALS SCIENCE AND ENGINEERING C, ELSEVIER SCIENCE S.A, CH, vol. 116, 18 June 2020 (2020-06-18), XP086247328, ISSN: 0928-4931, [retrieved on 20200618], DOI: 10.1016/J.MSEC.2020.111180
- ZHANG ZHINAN ET AL: "3D gel printing of porous calcium silicate scaffold for bone tissue engineering", JOURNAL OF MATERIAL SCIENCE, KLUWER ACADEMIC PUBLISHERS, DORDRECHT, vol. 54, no. 14, 17 April 2019 (2019-04-17), pages 10430 - 10436, XP036770179, ISSN: 0022-2461, [retrieved on 20190417], DOI: 10.1007/S10853-019-03626-1
- MA GUANSHENG ET AL: "Permeability and thermal expansion properties of porous LAS ceramic prepared by gel-casting method", JOURNAL OF THE EUROPEAN CERAMIC SOCIETY, ELSEVIER, AMSTERDAM, NL, vol. 40, no. 9, 31 March 2020 (2020-03-31), pages 3462 - 3468, XP086139144, ISSN: 0955-2219, [retrieved on 20200331], DOI: 10.1016/J.JEURCERAMSOC.2020.03.056
- ABREU JOÃO LUIZ BITTENCOURT DE ET AL: "Manufacturing and characterization of a 3D printed lithium disilicate ceramic via robocasting: A pilot study", JOURNAL OF THE MECHANICAL BEHAVIOR OF BIOMEDICAL MATERIALS, ELSEVIER, AMSTERDAM, NL, vol. 143, 27 April 2023 (2023-04-27), XP087331653, ISSN: 1751-6161, [retrieved on 20230427], DOI: 10.1016/J.JMBBM.2023.105867

## Beschreibung

### GEGENSTAND DER ERFINDUNG

Die Erfindung betrifft einen Satz von Chemikalien für die Herstellung einer Keramikdispersion oder eines Keramikgels, welche bzw. welches wiederum für die Herstellung von Keramikformteilen wie Keramikverblendungen oder Formkörpern wie Keramikteilkronen, Veneers, Keramikinlays und -onlays, vorzugsweise durch ein schichtweises Aufbauverfahren wie Stereolithographie, geeignet ist. Die Erfindung betrifft weiterhin ein Verfahren zur Herstellung eines Keramikformteils sowie ein Keramikformteil hergestellt durch dieses Verfahren.

### HINTERGRUND DER ERFINDUNG

Bei einem schichtweisen Aufbauverfahren für die Herstellung von keramischen Verblendungen oder Formkörpern wird ein Keramikgel oder eine Keramikdispersion mit einem (aushärtbaren) Polymermaterial schichtweise auf einen Grundkörper aufgetragen und gleichzeitig oder nachgelagert kontinuierlich getrocknet, beispielsweise durch Strahlungs- oder Wärmeeinwirkung. Hierdurch wird der sogenannte keramische "Grünling" erhalten. Anschließend erfolgt eine Entbinderung, d.h. ein Austreiben des in der Regel polymeren Bindemittels bei erhöhter Temperatur, wodurch der sogenannte "Weißling" erhalten wird. Bei dem Entbindern wird das Bindemittel durch thermische und/oder thermo-chemische Prozesse umgewandelt, die Wiederholungseinheit des Polymers zumindest teilweise verändert und zumindest teilweise zu flüchtigen Komponenten abgebaut.

Die Sinterung des Weißlings erfolgt im Sinterofen bei einem Hochtemperaturbrand. Dabei kommt es zur Verdichtung und Verfestigung des fein verteilten Keramikpulvers durch Temperatureinwirkung, wodurch das poröse Bauteil schrumpft und dessen Festigkeit zunimmt.

Schichtweise Aufbauverfahren sind jedoch stets mit dem Nachteil verbunden, dass bei Erhitzung, insbesondere im Schritt der Entbinderung, sich Fehlstellen wie Risse oder Blasen ausbilden können und dadurch das Bauteil unbrauchbar wird. Diesem Effekt kann meist nur dadurch begegnet werden, dass der Aufheizschritt verlangsamt wird, d.h. die Dauer der thermischen Behandlung erhöht wird, was jedoch das entsprechende Herstellverfahren unwirtschaftlich werden lässt.

Die US 5,496,682 offenbart beispielsweise aushärtbare Zusammensetzungen für die Herstellung dreidimensionaler Körper durch Stereolithographie, die 40 bis 70 Vol.-% Keramik- oder Metallpartikel, 10 bis 35 Gew.-% Monomer, 1 bis 10 Gew.-% Photoinitiator, 1 bis 10 Gew.-% Dispergiermittel und vorzugsweise auch Lösungsmittel, Weichmacher und Kopplungsmittel enthalten.

Aus der EP 2 233 449 A1 sind Schlicker zur Herstellung von Keramikformteilen durch Hot-Melt-Inkjet-Druckverfahren bekannt, die Keramikpartikel und mindestens ein radikalisch polymerisierbares Wachs enthalten und die Grünkörper ergeben, die sich im Wesentlichen ohne Rissbildung entbindern lassen. Bei stereolithographischen Verfahren müssen die Schlicker jedoch über längere Zeiträume in flüssiger Form stabil sein, d.h. insbesondere dürfen sich die im Schlicker dispergierten Partikel nicht vorzeitig absetzen, was im Hinblick auf den angestrebten möglichst hohen Volumenanteil der keramischen Partikel im Schlicker ein besonderes Problem darstellt.

Die EP 3 147 707 A1 offenbart ein stereolithographie-basiertes Verfahren, welches zumindest teilweise die oben beschriebenen Nachteile überwindet. Auch hier wird allerdings ein radikalischer Polymervorläufer zur Herstellung des Bindemittels eingesetzt.

Derartige Radikalreaktionen sind jedoch mit Nachteilen verbunden, denn die freien Radikale können die weiteren Bestandteile der Zusammensetzung, insbesondere bei Silikat-basierten Keramikgelen, schädigen, und dadurch zu Verfärbungen führen. Zudem wird bei radikalischen Reaktionen bei hohen Umsätzen eine exponentielle Steigerung der Reaktionsgeschwindigkeit beobachtet (Trommsdorff-Norrish-Effekt). Dieser Effekt ist auf die abnehmende Wahrscheinlichkeit eines Kettenabbruchs durch Rekombination der reaktiven Kettenenden zurückzuführen, welche wiederum durch die zunehmende Unbeweglichkeit der wachsenden Polymerketten hervorgerufen wird. Die damit zunehmende Geschwindigkeit der exothermen Reaktion bewirkt einen Temperaturanstieg, wodurch der Zerfall des Radikalstarters beschleunigt wird und die Konzentration der reaktiven Moleküle zunimmt. Gleichzeitig wird die Abfuhr der Reaktionswärme (Polymerisationswärme) durch die zunehmende Viskosität erschwert. In der Folge kann es zu lokalen Überhitzungen kommen, was bei einer schichtweise aufgetragenen Zusammensetzung zu Rissen, Explosionen oder einer Zersetzung der weiteren Inhaltsstoffe der keramischen Zusammensetzung führen kann. Auch die mechanischen Eigenschaften und die

Beständigkeit der aus den bekannten Zusammensetzungen erhaltenen Keramikformteile sind - insbesondere auch aufgrund der Anfälligkeit für Rissbildung und Zersetzung - eingeschränkt. Zudem weisen die keramischen Zusammensetzungen eine begrenzte Stabilität/Lagerungsfähigkeit auf.

### AUFGABE

Vor diesem Hintergrund bestand die Aufgabe der vorliegenden Erfindung daher darin, einen Satz von Chemikalien bereitzustellen, mit welchen die oben genannten Nachteile überwunden werden können. Der erfindungsgemäße Satz von Chemikalien soll ein komplikationsfreies Aufbauverfahren für Silikatmineral-basierte Keramikgele bzw. Keramikdispersionen ermöglichen, aus denen Keramikformteile mit hoher Qualität und geringer Verfärbung erhalten werden können.

### BESCHREIBUNG DER ERFINDUNG

Diese Aufgabe wird erfindungsgemäß gelöst durch einen Satz von Chemikalien für die Herstellung eines Keramikgels, umfassend nachfolgende Komponenten
A) ein vorzugsweise pulverförmiges Keramikmaterial, und
B) einen Binder, wobei
das Keramikmaterial ein Silikatkeramikmaterial ausgewählt aus der Gruppe bestehend aus Feldspat- und Leuzitkeramiken umfasst oder aus diesem besteht und der Binder einen Gelbildner umfasst oder aus diesem besteht, wobei der Gelbildner ein quervernetztes Polymer ist. Vorzugsweise handelt es sich bei dem quervernetzten Polymer um ein Polymer, das eine oder mehrere Wiederholungseinheiten aufweist, wobei mindestens eine der einen oder der mehreren Wiederholungseinheiten mindestens eine chelatisierende funktionelle Gruppe aufweist.

Der Satz von Chemikalien kann sich für die Herstellung einer Keramikdispersion, insbesondere einer Keramiksuspension und/oder eines Keramikgels, eignen. Hierzu kann dem Satz von Chemikalien vorzugsweise ein Löse- und/oder Dispersionsmittel wie Wasser oder ein Alkohol zugegeben werden bzw. weist dieser ein entsprechendes Löse- und/oder Dispersionsmittel auf. Bevorzugt ist diese Keramikdispersion für die Verwendung in stereolithographischen, Binder Jetting- oder Material Jetting-Verfahren geeignet.

Eine Dispersion ist in der Kolloidchemie und in der Verfahrenstechnik ein heterogenes Gemisch aus mindestens zwei Stoffen, die sich nicht oder kaum ineinander lösen oder chemisch miteinander verbinden. Dabei sind ein oder mehrere Stoffe als sogenannte disperse Phase fein verteilt in einem anderen kontinuierlichen Stoff, dem sogenannten Dispersionsmedium.

Ein "Gel" ist ein Beispiel eines dispersen Systems, das aus mindestens zwei Komponenten besteht. Die feste Komponente, der Gelbildner, bildet dabei mit seinen langen und/oder stark verzweigten Molekülen ein schwammartiges, dreidimensionales Netzwerk, dessen Poren durch eine Flüssigkeit (Lyogel) oder ein Gas (Xerogel) ausgefüllt sind. Die flüssige Komponente ist dadurch in der festen immobilisiert. Ist das Netzwerk hochporös und Luft das eingelagerte Gas, so wird das Gel auch als Aerogel bezeichnet.

Gelbildner verdicken als Gerüstbildner eine flüssige Phase und/oder bilden ein gummiartiges Gel aus. Daher sind sie auch als Verdickungsmittel oder zur Stabilisierung von Emulsionen einsetzbar. Es wird zwischen natürlichen (z. B. Agar-Agar), anorganischen (z. B. Bentonite), halbsynthetischen (z. B. Carboxymethylcellulose) und synthetischen Gelbildnern (z. B. Polyvinylalkohol) unterschieden. Die halbsynthetischen und synthetischen Gelbildnern sind aufgrund ihrer hohen Leistungsfähigkeit erfindungsgemäß besonders bevorzugt.

Unter "Löse- und/oder Dispersionsmittel" wird erfindungsgemäß eine unter Standardbedingungen (Temperatur: 298,15 K = 25°C, Druck: 1013,25 mbar = 1013,25 hPa) flüssige Substanz verstanden, in der sich die Bestandteile des Satzes von Chemikalien zumindest teilweise lösen und/oder dispergieren lassen.

Ein "Binder" ist erfindungsgemäß eine Substanz oder eine Kombination von Substanzen, die die Herstellung einer stabilen Dispersion oder eines Gels ermöglicht, indem sie die Keramikpartikel, insbesondere bei einer thermischen Behandlung, zusammenhält und stabilisiert sowie eine gleichförmige Verteilung ermöglicht. Als wesentliche Komponente weist der erfindungsgemäße Binder einen Gelbildner, beispielweise ein Polymer mit einer chelatisierenden funktionellen Gruppe, auf oder besteht aus diesem. Der erfindungsgemäße Binder trägt auch zur Stabilisierung des Keramikmaterials in feiner Verteilung in einem Löse- und/oder Dispersionsmittel bei, d.h. er erschwert die Sedimentation des Keramikmaterials oder verhindert diese sogar vollständig, sofern ein Löse- und/oder Dispersionsmittel eingesetzt wird.

Unter "Polymer" wird im erfindungsgemäßen Zusammenhang ein chemischer Stoff verstanden, welcher aus Makromolekülen besteht.

"Makromoleküle" sind Moleküle, die aus einer oder mehreren gleichen oder ähnlichen Struktureinheiten, den konstitutionellen Repetiereinheiten - auch Wiederholungseinheiten genannt -, aufgebaut sind *(*IUPAC. Compendium of Chemical Terminology, 2nd ed. (the "Gold Book"), A. D. McNaught, A. Wilkinson, Blackwell Scientific Publications, Oxford (1997), S. J. Chalk. ISBN 0-9678550-9-8*).* Solche Makromoleküle weisen mehr als 10 Repetiereinheiten, vorzugsweise mehr als 15 Repetiereinheiten auf. Die Molmasse beträgt vorzugsweise mindestens 3.000 g/mol, vorzugsweise mindestens 5.000 g/mol, besonders bevorzugt mindestens 7.000 g/mol und am bevorzugtesten mindestens 10.000 g/mol.

Polymere werden üblicherweise durch die Reaktion von Monomeren oder Oligomeren, die eine oder mehrere der konstitutionellen Wiederholungseinheiten aufweisen, in einer Polymerisationsreaktion hergestellt. Als Oligomer wird dabei ein Molekül bezeichnet, das aus mehreren Monomeren gebildet wurde und daher aus einer Vielzahl der strukturell gleichen oder ähnlichen Struktureinheiten aufgebaut ist. Von Oligomeren wird im Rahmen der Erfindung dann gesprochen, wenn das Molekül aus einer Reaktion von 2 bis 10, vorzugsweise 2 bis 8, vorzugsweise 3 bis 7 Monomeren hergestellt wurde.

Vorzugsweise weist der Binder über 50 Gew.-%, vorzugsweise über 70 Gew.-%, noch bevorzugter über 80 Gew.-%, noch stärker bevorzugt über 90 Gew.-% und am bevorzugtesten über 95 Gew.-% oder sogar 100 Gew.-% an Gelbildner, vorzugsweise in Form eines Polymers mit einer chelatisierenden funktionellen Gruppe, auf.

Vorzugsweise weist das Keramikmaterial über 50 Gew.-%, vorzugsweise über 70 Gew.-%, noch bevorzugter über 80 Gew.-%, noch stärker bevorzugt über 90 Gew.-% und am bevorzugtesten über 95 Gew.-% an Silikatkeramikmaterial auf.

Silikatkeramikmaterialien sind anorganische, nichtmetallische Werkstoffe, welche aus Silikat-Rohstoffen, d.h. Verbindungen mit [SiO₄]⁴⁻-Tetraeder in der Kristallstruktur, erhalten werden. Der SiO₂-Anteil der Silikatkeramikmaterialien ist vorzugsweise ≥ 20 Gew.-%, bevorzugter ≥ 30 Gew.-%. Vom Begriff Silikatkeramikmaterialien können auch Glaskeramiken umfasst sein. Vorzugsweise sind diese jedoch nicht umfasst. Vorzugsweise beträgt der Anteil von Glaskeramiken am Silikatkeramikmaterial weniger als 5 Gew.-%, bevorzugter weniger als 2 Gew.-%, noch stärker bevorzugt weniger als 1 Gew.-%, noch erheblich stärker bevorzugt weniger als 0,5 Gew.-% und am stärksten bevorzugt weniger als 0,1 Gew.-%.

Der Begriff "Satz von Chemikalien" wird erfindungsgemäß so verstanden, dass es sich um eine vorgegebene Zusammenstellung einzelner Chemikalien handelt, die entweder in separaten Gebinden oder teilweise bzw. vollständig vorgemischt in einer Zusammensetzung vorliegen. Besonders bevorzugt liegt der Satz von Chemikalien als Zusammensetzung, insbesondere als flüssige, pastöse oder feste Zusammensetzung, vor.

Unter "chelatisierende funktionelle Gruppen" werden funktionelle Gruppen verstanden, die zur Bildung von Chelaten befähigt sind. Chelate sind cyclische Koordinationsverbindungen, die mindestens ein zentrales Metallatom bzw. -ion und einen mehrzähnigen Liganden, den sogenannten Chelatliganden, aufweisen. Ein Ligand ist ein Ion oder Molekül, welches über eine koordinative Bindung an ein zentrales Metallatom bzw. -ion binden, d.h. koordinieren, kann. Die Abgrenzung der koordinativen Bindung zur klassischen kovalenten Bindung besteht darin, dass der Ligand bei der koordinativen Bindung beide Bindungselektronen zur Verfügung stellt, er ist daher eine "Lewis-Base". Mehrzähnig bedeutet in diesem Zusammenhang, dass der Ligand zwei oder mehr Koordinationsstellen aufweist, über die er an das Metallatom bzw. -ion koordiniert. In anderen Worten muss der Chelatligand also zwei oder mehr Atomgruppierungen aufweisen, die als Elektronendonatoren wirken können.

In einer bevorzugten Ausführungsform der Erfindung weist der Chelatligand drei oder mehr, bevorzugter vier oder mehr, besonders bevorzugt fünf oder mehr und am bevorzugtesten sechs oder mehr Koordinationsstellen auf.

Der Erfinder hat beobachtet, dass durch die Verwendung eines erfindungsgemäßen Gelbildners, beispielsweise eines Polymers mit einer chelatisierenden Gruppe, eine Silikatkeramikdispersion oder ein Silikatkeramikgel hergestellt werden kann, welche oder welches eine hohe Stabilität der Keramikpartikel aufweist, d.h. die Keramikpartikel keine oder nur eine sehr geringe Tendenz zur Segregation zeigen. Zudem weist die Zusammensetzung eine gut verarbeitbare, aber gleichzeitig ausreichend stabile Struktur auf. Dies war bisher nicht oder nur in unzureichendem Ausmaß möglich.

Insbesondere bei der Verwendung eines Polymers mit einer chelatisierenden Gruppe geht der Erfinder, ohne an diese Theorie gebunden zu sein, davon aus, dass die chelatisierenden Gruppen polare Wechselwirkungen mit den partiell positiv geladenen Siliziumatomen der Silikatkeramik ausbilden und hierdurch eine Stabilisierung, insbesondere in einer Dispersion, erzielt werden kann. Das Polymermaterial mit der mindestens einen chelatisierenden funktionellen Gruppe wirkt folglich als Binder. Da ein entsprechendes Polymermaterial gleichzeitig auch die Viskosität des Gels bzw. der Dispersion erhöht, wird ein fließfähiges und gut zu verarbeitendes, insbesondere spritzbares, Material erhalten. Hierdurch entfällt die Notwendigkeit, radikalisch polymerisierbare Polymervorläuferverbindungen in der Herstellung zu nutzen, um eine Verarbeitbarkeit entsprechender Keramikgele zu gewährleisten.

Mit dem erfindungsgemäßen Satz von Chemikalien lassen sich Keramikgele bzw. Keramikdispersionen herstellen, mit welchen auch äußerst filigrane und kleinteilige Strukturen konstruiert werden können. Zudem ist beim Entbinderungs- und Sinterungsprozess eine geringere Randschrumpfung zu beobachten, d.h. schwächer ausgeprägte Volumenänderungen. Der Erfinder geht davon aus, dass dies auf die gleichmäßige Verteilung der Keramikpartikel im Gel zurückzuführen ist. Zudem erfolgt die Entbinderung besonders effizient und schnell.

Um eine besonders gute Verarbeitbarkeit des Gels bzw. der Dispersion zu erreichen, weist dieses vorzugsweise eine erhöhte Viskosität auf. Um diese zu erzielen ist der erfindungsgemäße Gelbildner ein quervernetztes Polymer, genauer gesagt die Makromoleküle des Polymers des Satzes von Chemikalien sind quervernetzt.

Bevorzugte Gelbildner sind ausgewählt aus der Gruppe bestehend aus Polysacchariden wie Chitosan oder Chitosanderivaten, Alginsäuren, Xanthan oder Alginaten; Polyuronsäuren; Gelatine; Hyaluronsäure; Polyvinylalkoholen, Polyethylenglykolen; Ammonium Acryloyldimethyltaurate; Hydroxypropylstärke (HPS); Hydroxypropyldistärkephosphat (HDP); Polyquaternium, insbesondere 3-Methyl-1-vinylimidazolium methyl sulfate-N-vinylpyrrolidon copolymer oder Poly(2-methacryloxyethyltrimethylammonium chloride); Bentoniten; Sorbitanmonooleat; Polyethylenglycoltriether oder Polypropylenglycoltriether; Propylen Glycol Dicaprylate/Dicaprate; Silikatton und/oder Schichtsilikate wie Natrium-Magnesium Silikate oder Aluminium-Magnesium-Silikate, Natrium-Magnesium- oder Natrium-Magnesium-Fluor-Lithium-Schichtsilikate des Montmorillonit-Typs; Polyacrylamid; Kieselsäuren wie Aerosile; Sorbitan Oleat Decylglucosidcrosspolymere; Styrol-Maleinsäureanhydrid- oder Ethylen-Maleinsäureanhydrid-Copolymere und ihre Derivate; hydrophob modifizierte ethoxylierte Urethane; Polyvinylpyrrolidon; Amylopektin; Cellulose oder Cellulosederivaten wie Celluloseacetobutyrat; Polyacrylaten oder Polymethacrylaten sowie deren Ester, Copolymere und/oder Salze; oder Mischungen und Copolymere der vorgenannten. Bevorzugt sind die vorgenannten Gelbildner kovalent vernetzt.

Besonders bevorzugt handelt es sich bei dem Gelbildner um ein hydrogelbildendes Polymer. Ein Hydrogel ist ein Gel aus einem wasserunlöslichen Polymer, das Wasser binden kann. Die Moleküle, die das Gel aufbauen, sind chemisch, z. B. durch kovalente, supramolekulare oder ionische Bindungen, oder physikalisch, z. B. durch Verschlaufungen der Polymerketten, zu einem Netzwerk verknüpft, welches das Lösemittel wie Wasser binden kann. Kovalent vernetzte Polymere sind zum Beispiel thiolisierte Polymere (Thiomere), die durch die Ausbildung von Disulfidbrücken Polymerketten vernetzen.

Eine bevorzugte Sonderform der Gelbildnern sind superabsorbierende Polymere. Superabsorbierende Polymere ("Superabsorber") sind vorzugsweise vernetzte Polymere, die in der Lage sind, ein Vielfaches ihres Eigengewichts an Lösemitteln, insbesondere polaren Lösemitteln wie Wasser, aufzunehmen. Bei der Aufnahme der Flüssigkeit quillt der Superabsorber auf und bildet ein Hydrogel. Die Funktionsweise von superabsorbierenden Polymeren wird beispielsweise in Koltzenburg, S., Nuyken, O., Maskos, M. (2013). Polymere: Synthese, Eigenschaften und Anwendungen. Deutschland: Springer Berlin Heidelberg beschrieben.

Erfindungsgemäß bevorzugt ist das superabsorbierende Polymer ein vorzugsweise quervernetztes Copolymer mit einer Acrylsäure und/oder Natriumacrylat und/oder Acrylsäureester Wiederholungseinheit.

Erfindungsgemäß bevorzugt ist das superabsorbierende Polymer ein vorzugsweise quervernetztes Copolymer mit einer Methacrylsäure und/oder Natriummethacrylat und/oder Methacrylsäureester Wiederholungseinheit.

Besonders bevorzugt ist ein quervernetztes Copolymer aus Acrylsäure und Natriumacrylat oder ein quervernetztes Copolymer aus Acrylsäure und/oder Natriumacrylat und/oder Acrylamid, insbesondere aus Acrylsäure und Natriumacrylat und Acrylamid.

Besonders bevorzugt ist ein quervernetztes Copolymer aus Methacrylsäure und Natriummethacrylat oder ein quervernetztes Copolymer aus Methacrylsäure und/oder Natriummethacrylat und/oder Methacrylamid, insbesondere aus Methacrylsäure und Natriummethacrylat und Methacrylamid. Besonders bevorzugt sind entsprechende Copolymere mit einem Allylether von Saccharose oder einem Arylether von Pentaerythritol quervernetzt.

Besonders bevorzugt sind - insbesondere aufgrund ihrer Anwendungsbreite und guten Verfügbarkeit - Carbomere, insbesondere solche, welche mit Polyalkoholen, Polyalkenethern wie Polyalkoholallylethern oder Polyalkenethern von Zuckern, Allylether-Saccharose oder Allylether-Pentaerythritol, quervernetzt wurden.

Als besonders bevorzugt haben sich in der Anwendung zudem solche Polymere erwiesen, deren chelatisierende Gruppe ein oder mehrere Sauerstoffatome umfassen. Die Erfinder gehen davon aus, dass aufgrund der besonders ausgeprägten Si-O-Wechselwirkung der Stabilisierungseffekt besonders hoch ist. Die chelatisierende Gruppe kann beispielsweise eine Säure, Salz oder Estergruppe wie beispielsweise in einer Polyacrylsäure oder einem Polyacrylat sein. Als besonders bevorzugt haben sich ebenfalls Polyvinylpyrrolidon oder Polyvinylphosphonate erwiesen. Besonders bevorzugt sind die Makromoleküle der vorgenannten Polymere quervernetzt, vorzugsweise handelt es sich hierbei um eine kovalente Quervernetzung. Die Begriffe "vernetzt" und "quervernetzt" werden vorliegend synonym verwendet und bezeichnen die Verknüpfung einer Vielzahl von Makromolekülen des Polymers zu einem dreidimensionalen Netzwerk. Die Vernetzung kann direkt beim Aufbau der Makromoleküle oder durch Reaktionen an bereits bestehenden Polymeren erreicht werden. Hierdurch wird ein Aufquellen des Polymers beobachtet, welches wiederum die Dispersionsvermittlung positiv beeinflusst.

Der Satz von Chemikalien weist vorzugsweise weniger als 5 Gew.-%, bevorzugter weniger als 2 Gew.-%, noch stärker bevorzugt weniger als 1 Gew.-%, noch erheblich stärker bevorzugt weniger als 0,5 Gew.-% und am stärksten bevorzugt weniger als 0,1 Gew.-% an polymerisierbaren Polymervorläuferverbindungen wie Monomere oder Oligomere auf.

Besonders bevorzugt beträgt das Gewichtsverhältnis von polymerisierbaren Polymervorläuferverbindungen zu Polymer im Satz von Chemikalien 1:10 oder größer, vorzugsweise 1:50 oder größer und besonders bevorzugt 1:100 und größer.

Durch einen entsprechend niedrigen Anteil von polymerisierbaren Verbindungen werden Neben- und Zersetzungsreaktionen bei der Herstellung des Gels vermieden.

Bevorzugt weist der Binder und/oder der Gelbildner einen geringen Veraschungsrückstand auf, vorzugsweise ≤ 10 Gew.-%, bevorzugter ≤ 5 Gew.-%, noch stärker bevorzugt ≤ 1 Gew.-%, noch erheblich stärker bevorzugt ≤ 0,5 Gew.-%, und am bevorzugtesten ≤ 0,2 Gew.-%.

Die Bestimmung des Veraschungsrückstands, also des nach thermischer Behandlung der Veraschung verbliebenen Rückstands, kann wie in DIN EN ISO 3451-1 beschrieben, insbesondere mit Hilfe thermogravimetrischer Analyse, erfolgen. Ein geringer Veraschungsrückstand sorgt für einen geringeren Verfärbungsgrad des Keramikformteils.

Der erfindungsgemäße Satz von Chemikalien weist vorzugsweise weiterhin ein Löse- und/ oder Dispersionsmittel auf. In diesem Zusammenhang sind insbesondere polare und/oder protische Lösemittel wie Wasser und/oder Alkohole, insbesondere mehrwertige Alkohole wie Ethylenglykol oder Monopropylenglykol, bevorzugt. Diese sind besonders dafür geeignet, die Komponenten des aus dem Satz von Chemikalien zu erhaltenen Gels bzw. der Dispersion in dispergierter oder gelöster Form zu halten und hierdurch die Verarbeitbarkeit des Gels bzw. der Dispersion zu ermöglichen.

In einer bevorzugten Ausführungsform der Erfindung beträgt der Gewichtsanteil des Löse- und/oder Dispersionsmittel an dem Gewicht des Satzes von Chemikalien mindestens 5 Gew.-%, vorzugsweise mindestens 10 Gew.-%, noch bevorzugter mindestens 15 Gew.-%, noch stärker bevorzugt mindestens 20 Gew.-%, noch erheblich stärker bevorzugt mindestens 30 Gew.-% und am bevorzugtesten mindestens 40 Gew.-%, jedoch in der Regel ≤ 70 Gew.-%.

Bevorzugt beträgt der Gewichtsanteil des Löse- und/oder Dispersionsmittel an dem Gewicht des Satzes von Chemikalien ohne Einbeziehung des Gewichtes des Keramikmaterials 50 Gew.-% bis 99,9 Gew.-%, bevorzugter 70 Gew.-% bis 99 Gew.-% und am bevorzugtesten 90 Gew.-% bis 99 Gew.-%.

In einer bevorzugten Ausführungsform der Erfindung beträgt der Gewichtsanteil des Keramikmaterials an dem Gewicht des Satzes von Chemikalien ≤ 70 Gew.-%, vorzugsweise ≤ 50 Gew.-%, noch bevorzugter ≤ 40 Gew.-%, noch stärker bevorzugt ≤ 35 Gew.-% und am bevorzugtesten ≤ 30 Gew.-%, jedoch vorzugsweise ≥ 20 Gew.-%.

In einer bevorzugten Ausführungsform der Erfindung liegt der Gewichtsanteil des Keramikmaterials an dem Gewicht des Satzes von Chemikalien im Bereich von 5-70 Gew.-%, vorzugsweise 10-50 Gew.-%, noch stärker bevorzugt 15-40 Gew.-% und am bevorzugtesten 20-35 Gew.-%.

Durch entsprechend niedrige Anteile von Keramikmaterial kann in der Regel ein gut fließfähiges und damit gut handhabbares Gel hergestellt werden.

Besonders bevorzugt umfasst die Zusammensetzung weiterhin eine oder mehrere Basen, insbesondere eine Brønsted-Base, d.h. eine Verbindung, deren einmolare wässrige Lösung einen pH-Wert >7, vorzugsweise >8, aufweist. Eine Base sorgt dafür, dass eine verbesserte Wechselwirkung zwischen Keramikpartikel und Polymermaterial erzeugt wird. Insbesondere bei chelatisierenden Gruppen mit Säurefunktion dient die Base zum Abfangen des Protons, wodurch die Komplexbildungsstärke der chelatisierenden Gruppe weiter verstärkt wird. Dieser Effekt ist insbesondere bei Polyacrylsäuren bzw. Polyacrylaten und Polyvinylphosphonsäuren bzw. Polyvinylphosphonaten zu beobachten. Besonders bevorzugt ist die Base ausgewählt aus der Gruppe bestehend aus Alkali- und Erdalkalimetallhydroxiden und Alkali- und Erdalkalicarbonaten. Besonders bevorzugt ist die Base ausgewählt aus der Gruppe bestehend aus NaOH, KOH, Na₂CO₃, K₂CO₃, CaCO₃, CaO und Ca(OH)₂, NaHCO₃ oder Mischungen der vorgenannten.

Der Satz von Chemikalien weist vorzugsweise ≤15 Gew.-%, bevorzugter weniger als 10 Gew.-%, noch stärker bevorzugt ≤5 Gew.-%, noch erheblich stärker bevorzugt ≤1 Gew.-% und am bevorzugtesten ≤ 0,5 Gew.-% an Basen auf. Der Mindestgehalt an Basen beträgt vorzugsweise ≥ 0,05 Gew.-%, bevorzugter ≥ 0,1 Gew.-%.

Bevorzugt beträgt der Gewichtsanteil der Base(n) an dem Gewicht des Satzes von Chemikalien ohne Einbeziehung des Gewichtes des Keramikmaterials 0,10 Gew.-% bis 0,99 Gew.-%, bevorzugter 0,20 Gew.-% bis 0,8 Gew.-% und am bevorzugtesten 0,3 Gew.-% bis 0,7 Gew.-%.

In einer bevorzugten Ausführungsform der Erfindung weist der Satz von Chemikalien als zusätzliche Komponenten ein Konservierungsmittel auf, das vorzugsweise biozid ist. Konservierungsmittel dienen dazu, die Haltbarkeit des Keramikgels bzw. der Keramikdispersion zu erhöhen, insbesondere indem sie Zersetzungsprozesse verhindern. Bevorzugt werden als Konservierungsmittel schwefelhaltige Konservierungsmittel, insbesondere Isothiazolinone, eingesetzt. Durch die bakterizide und fungizide Wirkung schützen sie vor einer mikrobiellen Zersetzung der Komponenten des Satzes von Chemikalien. Besonders bevorzugt sind 5-chlor-2-Methyl-4-Isothiazolin-3-one, 2-Methyl-4-Isothiazolin-3-one, Isothiazolinone-3-one, Methylisothiazolinon, Benzisothiazolinon, Octylisothiazolinon, Dichloroctylisothiazolinon oder Mischungen der vorgenannten. Alternativ sind auch Formaldehydabspalter wie O-Formale oder N-Formale als Konservierungsmittel geeignet. Besonders bevorzugt sind Benzylhemiformal, 1,6-Dihydroxy-2,5-dioxahexan, Methylolharnstoff, 7-Ethylbicyclooxazolidin, Methenamin, Paraformaldehyd, Tris(hydroxymethyl)nitromethan oder Mischungen der vorgenannten.

Der Satz von Chemikalien weist vorzugsweise ≤5 Gew.-%, bevorzugter ≤2 Gew.-%, noch stärker bevorzugt ≤1 Gew.-%, noch erheblich stärker bevorzugt ≤0,5 Gew.-% und am bevorzugtesten ≤ 0,25 Gew.-% an Konservierungsmitteln auf. Der Mindestgehalt an Konservierungsmitteln beträgt vorzugsweise ≥ 0,05 Gew.-%, bevorzugter ≥ 0,1 Gew.-%.

Um die Farbgebung des Keramikgels bzw. der Keramikdispersion zu steuern, kann der Satz von Chemikalien weiterhin eine farbgebende Komponente aufweisen, die vorzugsweise ausgewählt ist aus Übergangsmetallverbindungen, insbesondere Oxiden wie Er₂O₃, Fe₂O₃, Co₃O₄, MnO₂, NiO₂, Cr₂O₃,Pr₂O₃, Tb₂O₃, Bi₂O₃ und Mischungen der vorgenannten. Alternativ oder zusätzlich können jedoch auch Acetylacetonate oder Carbonsäuresalze von Eisen, Cer, Praseodym, Nickel, Terbium, Lanthan, Wolfram, Osmium, Terbium und Mangan eingesetzt werden.

Die farbgebenden Komponenten sind vorzugsweise so gewählt, dass nach dem Entbindern und Sintern zahnfarbene Keramikformteile erhalten werden können.

Die farbgebenden Komponenten könnten vorzugsweise auch in dem Keramikmaterial enthalten sein. Das Keramikmaterial kann auch weitere Additive wie Fließ- oder Trennmittel enthalten.

Der Satz von Chemikalien weist vorzugsweise weniger als 5 Gew.-%, bevorzugter ≤2 Gew.-%, noch stärker bevorzugt ≤1 Gew.-%, noch erheblich stärker bevorzugt ≤0,5 Gew.-% und am bevorzugtesten ≤ 0,25 Gew.-% an den obigen farbgebenden Komponenten auf. Der Mindestgehalt an den obigen farbgebenden Komponenten beträgt vorzugsweise ≥ 0,05 Gew.-%, bevorzugter ≥ 0,1 Gew.-%.

In einer bevorzugten Ausführungsform der Erfindung beträgt der Gewichtsanteil des Keramikmaterials an dem Gewicht des Satzes von Chemikalien mindestens 40 Gew.-%, vorzugsweise mindestens 50 Gew.-%, noch bevorzugter mindestens 55 Gew.-%, noch stärker bevorzugt mindestens 60 Gew.-% und am bevorzugtesten mindestens 65 Gew.-%.

In einer bevorzugten Ausführungsform der Erfindung beträgt der Gewichtsanteil des Keramikmaterials an dem Gewicht des Satzes von Chemikalien maximal 95 Gew.-%, vorzugsweise maximal 90 Gew.-%, noch bevorzugter maximal 85 Gew.-%, noch stärker bevorzugt maximal 80 Gew.-% und am bevorzugtesten maximal 75 Gew.-%.

In einer bevorzugten Ausführungsform der Erfindung liegt der Gewichtsanteil des Keramikmaterials an dem Gewicht des Satzes von Chemikalien im Bereich von 50 bis 90 Gew.-%, vorzugsweise 55 bis 85 Gew.-%, noch stärker bevorzugt 60 bis 80 Gew.-% und am bevorzugtesten 65 bis 75 Gew.-%.

Durch entsprechend hohe Anteile von Keramikmaterial kann in der Regel ein sehr kompaktes Gel mit geringem Veraschungsrückstand erhalten werden.

Durch einen entsprechend hohen Anteil von keramischem Material wird zudem die Wahrscheinlichkeit für mechanische Beschädigungen bei der thermischen Behandlung des Keramikgels bzw. der Dispersion deutlich reduziert und ein homogeneres Keramikformteil erhalten.

In einer bevorzugten Ausführungsform der Erfindung beträgt der Gewichtsanteil des Gelbildners, beispielsweise in Form eines Polymers, an dem Gewicht des Satzes von Chemikalien ≥ 0,1 Gew.-%, stärker bevorzugt ≥ 0,2 Gew.-%, noch stärker bevorzugt ≥0,5 Gew.-%, noch stärker bevorzugt mindestens 1 Gew.-%, noch wesentlich stärker bevorzugt mindestens 1,5 Gew.-%, noch erheblich bevorzugter mindestens 2 Gew.-%, und am bevorzugtesten mindestens 2,5 Gew.-%. Hierdurch wird eine besonders ausgeprägte Stabilisierung des Keramikmaterials im Keramikgel bzw. der Keramikdispersion bewirkt. Bevorzugt ist der Gewichtsanteil des Polymers jedoch nicht höher als 3,5 Gew.-% oder 4 Gew.-%, sodass der einer thermischen Behandlung des Satz von Chemikalien erhaltene Veraschungsrückstand möglichst gering ist Bevorzugt beträgt der Gewichtsanteil des Gelbildners an dem Gewicht des Satzes von Chemikalien ohne Einbeziehung des Gewichtes des Keramikmaterials 0,10 Gew.-% bis 0,99 Gew.-%, bevorzugter 0,20 Gew.-% bis 0,8 Gew.-% und am bevorzugtesten 0,3 Gew.-% bis 0,7 Gew.-%.

Das Gewichtsverhältnis von Keramikmaterial zu dem Gewicht der übrigen Komponenten des Satzes von Chemikalien liegt vorzugsweise im Bereich von 1:1 bis 5:1, bevorzugter 2:1 bis 4:1 und am bevorzugtesten 3:1 bis 4:1. Sehr bevorzugt sind auch Verhältnisse von Keramikpulver zu Binder von 60:40 bis 70:30.

Die Silikatkeramik ist ausgewählt aus der Gruppe bestehend aus Feldspat- und Leuzitkeramiken.

Besonders bevorzugt sind Feldspatkeramiken, insbesondere in Kombination mit Polyacrylsäure und/oder einem Polyacrylat als Polymer. Bei dieser Kombination wurde ein besonders ausgeprägter Stabilisierungseffekt beobachtet.

Die vorliegende Erfindung betrifft auch eine Zusammensetzung, insbesondere ein Gel oder eine Dispersion, bestehend aus oder enthaltend den Satz von Chemikalien. Vorzugsweise beträgt der Gewichtsanteil des Satzes von Chemikalien an dem Gesamtgewicht der Zusammensetzung 10 bis 70 Gew.-%, vorzugsweise 15 bis 70 Gew.-%, noch bevorzugter 20 bis 60 Gew.-%, noch stärker bevorzugt 25 bis 60 Gew-% und am bevorzugtesten 30 bis 60 Gew.-%.

Die Herstellung des Gels oder der Dispersion erfolgt durch vorzugsweise mechanisches Vermischen der Komponente B), d.h. des Binders, und optional enthaltenen Bestandteilen, wie C) oder E), mit dem Keramikmaterial, das vorzugsweise in Form eines Keramikpulvers vorliegt.

Die Erfindung betrifft auch ein Verfahren zur Herstellung eines Keramikformteils umfassend oder bestehend aus nachfolgenden Schritten
a₁) Mischen der Komponenten eines Satzes von Chemikalien, wie oben definiert, um ein Gel oder eine Dispersion zu erhalten,
b) Bestrahlung oder Wärmebehandlung des Gels oder der Dispersion in einem Temperaturbereich von 50° bis 150° C, um einen Grünling zu erhalten,
c) zumindest teilweise, vorzugsweise vollständige Entfernung des Bindemittels, vorzugsweise durch Wärmebehandlung des Grünlings in einem Temperaturbereich von 150° bis 350°C oder durch Bestrahlung, um einen Weißling zu erhalten,
d) Sintern des Weißlings oder wenn Schritte b) und/oder c) optional sind des Grünlings oder des Gels oder der Dispersion durch Wärmebehandlung in einem Temperaturbereich von 700 °C bis 1000 °C, um das Keramikformteil zu erhalten.

Die Verfahrensschritte werden vorzugsweise voneinander zeitlich getrennt in der Reihenfolge a₁) bis d) durchgeführt.

Obige Schritte b) und c) sind optional und können zusammen, d.h. in einem Schritt ohne zeitliche Trennung, durchgeführt werden. Die Herstellung des Keramikformteils kann auch ohne die Zwischenschritte der Herstellung eines Grünlings bzw. Weißlings erfolgen. Die Entfernung des Bindemittels erfolgt dann in Schritt d). Eine besonders hohe Produktqualität wird jedoch bei Durchführung dieser Schritte erhalten.

Das erfindungsgemäße Verfahren zur Herstellung eines Keramikformteils umfasst vorzugsweise nachfolgenden zusätzlichen Schritt:
a₂) schichtweises Auftragen des Gels oder der Dispersion auf eine Oberfläche, vorzugsweise auf eine Metall- oder Zirkonkappe oder einen Einbettmassestumpf, wobei das Auftragen vorzugsweise durch Spritztechnik und/oder ein 3D-Druck-Verfahren, besonders bevorzugt mittels Multi-Jet Modeling, erfolgt.

Dieser Schritt wird vorzugsweise zwischen Schritt a₁) und b) durchgeführt und zwar besonders bevorzugt zeitlich getrennt von diesen Schritten.

Vorzugsweise werden die Schritte a₂) und/oder b) und/oder c) und/oder d) des erfindungsgemäßen Verfahrens in einem Rapid-Prototyping-Verfahren, insbesondere einem Stereolithographieverfahren, durchgeführt.

Die Erfindung betrifft auch ein Keramikformteil, wie eine Krone, ein Kronenteil, ein Veneer, ein Keramikinlay oder -onlay, eine Brückenankerkrone, Brückenzwischenglieder sowie Teile der vorgenannten, welches durch das erfindungsgemäße Verfahren hergestellt wurde. Durch den Einsatz des erfindungsgemäßen Satzes von Chemikalien im oben dargestellten Verfahren werden Keramikformteile mit besonders hoher Homogenität und natürlichem Aussehen erhalten. Sie zeigen daher bevorzugte Eigenschaften gegenüber den aus dem Stand der Technik bekannten Keramikformteilen. Die Entfernung des Bindemittels, vorzugsweise durch Wärmebehandlung des Grünlings in einem Temperaturbereich von 150° bis 350°C oder durch Bestrahlung, insbesondere Infrarotlichtbestrahlung, um einen Weißling zu erhalten, erfolgt mit dem erfindungsgemäßen Gel bzw. der erfindungsgemäßen Dispersion besonders schnell und homogen. In dem oben genannten Temperaturbereich können Entbinderungszeiten von ≤ 20 Minuten, ≤ 15 Minuten oder sogar ≤ 10 Minuten erzielt werden.

Die nach dem erfindungsgemäßen Verfahren hergestellten Keramikformkörper zeichnen sich zudem durch eine hohe Festigkeit und große Detailgenauigkeit aus. Die Biegefestigkeit nach ISO 6872 liegt bei Formkörpern aus Feldspat- oder Glaskeramik vorzugsweise über 50 MPa, insbesondere im Bereich von 100 bis 500 MPa.

Die vorliegende Erfindung betrifft weiterhin die Verwendung einer Kombination aus einer Silikatkeramik ausgewählt aus der Gruppe bestehend aus Feldspat- und Leuzitkeramiken und einem quervernetzten Polymer, in einem Gel zur Herstellung einer Keramikverblendung.

Die Erfindung betrifft auch die Verwendung eines Gelbildners als Binder in einem Gel bzw. einer Dispersion zur Herstellung eines Keramikformteils, wobei das Gel vorzugsweise eine Silikatkeramik umfasst oder aus dieser besteht.

Die Erfindung betrifft auch die Verwendung wie in Anspruch 13 definiert.

### BEISPIELE

Die Erfindung wird nun anhand eines explizierten Ausführungsbeispiels nebst entsprechender photographischer Darstellungen beispielhaft beschrieben.

### HERSTELLUNGSBEISPIEL 1

In einer Petrischale wird eine Mischung der nachfolgenden Komponenten hergestellt:

| | |
|---|---|
| 1) | entionisiertes Wasser (~70 Gew.-%) |
| 2) | Carbomer (~17 Gew.-%) |
| 3) | Natriumhydroxid (~3 Gew.-%) |
| 4) | Monopropylenglykol (~ 7 Gew.-%) |
| 5) | Mischung aus 5-chlor-2-Methyl-4-Isothiazolin-3-one, 2-Methyl-4-Isothiazolin-3-one, Isothiazolinone-3-one (Rest) |

Nach Erhalt einer homogenen Paste wird zu dieser Mischung ein Feldspat- oder Glaskeramikpulver zugemischt. In Bezug auf die Summe der Massen der Mischung mit den Komponenten 1) bis 5) wird die 3-4-fache Menge an Keramikpulver zugegeben, um nach mechanischem Vermischen eine spritzbare Keramikdispersion zu erhalten. Die Masse wird in eine 10 ml Spritze der Firma Coltene überführt und damit schichtweise auf einen Gerüstkörper aufgebracht. Es folgt eine Wärmebehandlung bei 150°C, um einen Grünling zu erhalten. Durch anschließende Wärmebehandlung bei 300 °C wird ein Weißling erhalten, der anschließend bei 950 °C gebrannt wird.

### HERSTELLUNGSBEISPIEL 2

In einer Petrischale wird eine Mischung der nachfolgenden Komponenten hergestellt:

| | |
|---|---|
| 1) | entionisiertes Wasser (~98,5 Gew.-%) |
| 2) | Carbomer (~1,0 Gew.-%) |
| 3) | Kaliumhydroxid (~0,5 Gew.-%) |

Nach Erhalt einer homogenen Paste wird zu dieser Mischung ein Feldspat- oder Glaskeramikpulver zugemischt. In Bezug auf die Summe der Massen der Mischung mit den Komponenten 1) bis 3) wird die 3-4-fache Menge an Keramikpulver zugegeben, um nach mechanischem Vermischen eine spritzbare Keramikdispersion zu erhalten. Die Masse wird in eine 10 ml Spritze der Firma Coltene überführt und damit schichtweise auf einen Gerüstkörper aufgebracht. Es folgt eine Wärmebehandlung bei 150°C, um einen Grünling zu erhalten. Durch anschließende Wärmebehandlung bei 300 °C wird ein Weißling erhalten, der anschließend bei 950 °C gebrannt wird.

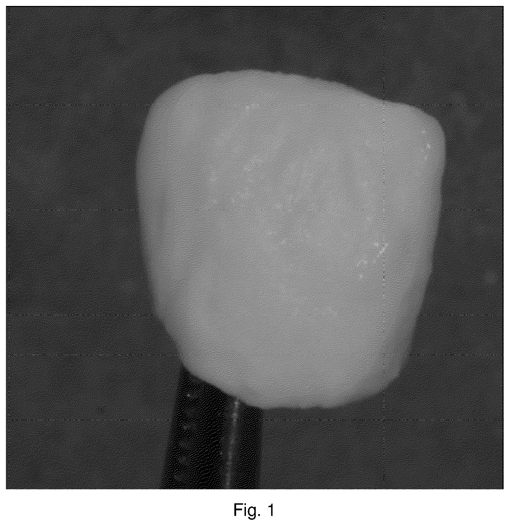

Fig. 1 zeigt eine photographische Darstellung des durch das obige Verfahren "Herstellungsbeispiel 1" erhaltenen Weißlings vor dem Brand.

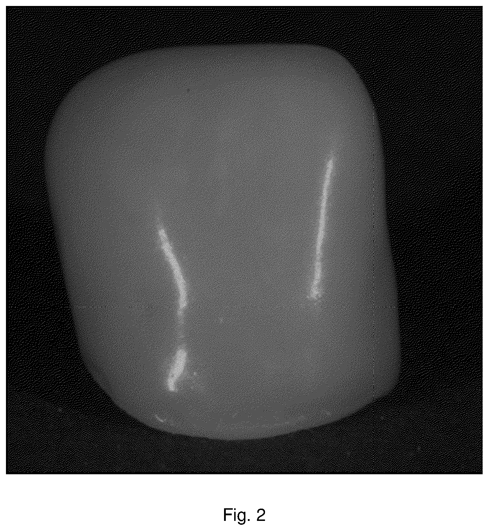

Fig. 2 zeigt denselben Weißling nach dem Brennen bei 950 °C für 60 min. Wie aus der Fig. 2 ersichtlich, wird ein gleichförmiges Keramikteil mit geschlossener Struktur erhalten.

## Patentansprüche

1. **Satz von Chemikalien,** umfassend nachfolgende Komponenten
A) ein Keramikmaterial, das ein Silikatkeramikmaterial umfasst oder aus diesem besteht,
B) einen Binder, der einen Gelbildner umfasst oder aus diesem besteht, wobei der Gelbildner ein quervernetztes Polymer ist,
**dadurch gekennzeichnet, dass**
das Silikatkeramikmaterial ausgewählt ist aus der Gruppe bestehend aus Feldspat- und Leuzitkeramiken.

2. **Satz von Chemikalien** nach Anspruch 1, wobei das Polymer des Gelbildners eine oder mehrere Wiederholungseinheiten umfasst, wobei vorzugsweise mindestens eine der einen oder mehreren Wiederholungseinheiten eine chelatisierende funktionelle Gruppe aufweist.

3. **Satz von Chemikalien** nach Anspruch 1 oder 2, wobei das Polymer ein superabsorbierendes Polymer, vorzugsweise ein Copolymer mit einer (Meth)acrylsäure und/oder Natrium(meth)acrylat-Wiederholungseinheit, ist.

4. **Satz von Chemikalien** nach einem der vorausgehenden Ansprüche, wobei der Satz nachfolgende zusätzliche Komponente enthält:
C) ein Löse- und/oder Dispersionsmittel, das vorzugsweise ausgewählt ist aus der Gruppe bestehend aus Wasser und Alkoholen.

5. **Satz von Chemikalien** nach einem der vorausgehenden Ansprüche, wobei der Satz nachfolgende zusätzliche Komponente enthält:
D) eine Base, die vorzugsweise ausgewählt ist aus der Gruppe bestehend aus Alkali- und Erdalkalimetallhydroxiden und Alkali- und Erdalkalicarbonaten.

6. **Satz von Chemikalien** nach einem der vorausgehenden Ansprüche, wobei der Satz nachfolgende zusätzliche Komponente enthält:
E) ein vorzugsweise biozides Konservierungsmittel.

7. Satz von Chemikalien nach einem der vorausgehenden Ansprüche, wobei der Satz nachfolgende zusätzliche Komponente enthält:
F) eine farbgebende Komponente, die vorzugsweise ausgewählt ist aus Er₂O₃, Fe₂O₃, Co₃O₄, MnO₂, NiO₂, Cr₂O₃, Pr₂O₃, Tb₂O₃, Bi₂O₃ und Mischungen der vorgenannten.

8. Satz von Chemikalien nach einem der vorausgehenden Ansprüche, wobei der Gewichtsanteil des Keramikmaterials an dem Gewicht des Satzes von Chemikalien im Bereich von 50 bis 90 Gew.-%, vorzugsweise 60 bis 80 Gew.-% liegt.

9. Gel oder Dispersion umfassend den Satz von Chemikalien wie in einem der vorausgehenden Ansprüche definiert.

10. Verfahren zur Herstellung eines Keramikformteils umfassend nachfolgende Schritte
a₁) Mischen der Komponenten eines Satzes von Chemikalien, wie er in einem der vorausgehenden Ansprüche definiert ist, um ein Gel oder eine Dispersion zu erhalten,
b) Optional: Wärmebehandlung des Gels oder der Dispersion in einem Temperaturbereich von 50° C bis 200° C, um einen Grünling zu erhalten,
c) Optional: Entfernung des Binders durch Wärmebehandlung des Grünlings in einem Temperaturbereich von 250° C bis 350° C, um einen Weißling zu erhalten,
d) Sintern durch Wärmebehandlung in einem Temperaturbereich von 700 °C bis 1000 °C, um das Keramikformteil zu erhalten.

11. Verfahren zur Herstellung eines Keramikformteils nach Anspruch 10 umfassend nachfolgenden zusätzlichen Schritt
a₂) schichtweises Auftragen des Gels oder der Dispersion auf eine Oberfläche, vorzugsweise durch Spritztechnik und/oder ein 3D-Druck-Verfahren, besonders bevorzugt mittels Binder Jetting, Material Jetting, Poly-Jet- oder Multi-Jet Modeling.

12. **Keramikformteil** erhältlich durch ein Verfahren wie in den Ansprüchen 10 oder 11 definiert.

13. **Verwendung** einer Kombination aus einem Silikatkeramikmaterial ausgewählt aus der Gruppe bestehend aus Feldspat- und Leuzitkeramiken und einem Binder umfassend einen Gelbildner mit quervernetzten Polymer in einem Gel oder einer Dispersion, zur Herstellung einer Keramikverblendung.

## Claims

1. **Set of chemicals** comprising the following components
A) a ceramic material comprising or consisting of a silicate ceramic material,
B) a binder comprising or consisting of a gelling agent, wherein the gelling agent is a crosslinked polymer,
**characterized in that**
the silicate ceramic material is selected from the group consisting of feldspar and leucite ceramics.

2. **Set of chemicals** according to claim 1, wherein the polymer of the gelling agent comprises one or more repeating units, wherein preferably at least one of the one or more repeating units has a chelating functional group.

3. **Set of chemicals** according to one of claims 1 or 2, wherein the polymer is a superabsorbent polymer, preferably a copolymer with a (meth)acrylic acid and/or sodium (meth)acrylate repeating unit.

4. **Set of chemicals** according to any one of the preceding claims, wherein the set comprises the following additional component:
C) a solvent and/or dispersing agent preferably selected from the group consisting of water and alcohols.

5. **Set of chemicals** according to any one of the preceding claims, wherein the set contains the following additional component:
D) a base, preferably selected from the group consisting of alkali metal and alkaline earth metal hydroxides and alkali metal and alkaline earth metal carbonates.

6. **Set of chemicals** according to any of the preceding claims, wherein the set contains the following additional component:
E) a preferably biocidal preservative.

7. **Set of chemicals** according to any one of the preceding claims, wherein the set contains the following additional component:
F) a coloring component preferably selected from Er₂O₃, Fe₂O₃, Co₃O₄, MnO₂, NiO₂, Cr₂O₃, Pr₂O₃, Tb₂O₃, Bi₂O₃ and mixtures of the foregoing.

8. **Set of chemicals** according to any one of the preceding claims, wherein the weight proportion of the ceramic material in relation to the weight of the set of chemicals is in the range of 50 to 90 wt.%, preferably 60 to 80 wt.%.

9. **Gel or dispersion** comprising the set of chemicals as defined in any of the preceding claims.

10. **Process** for the preparation of a ceramic molded part comprising the following steps
a₁) mixing the components of a set of chemicals as defined in any one of the preceding claims to obtain a gel or dispersion,
b) optionally: heat treating the gel or dispersion in a temperature range of from 50° C to 200° C to obtain a green body,
c) optionally: removal of the binder by heat treatment of the green body in a temperature range of from 250° C to 350° C to obtain a white body,
d) sintering by heat treatment in a temperature range of from 700°C to 1000°C to obtain the ceramic molded part.

11. **Process** of manufacturing a ceramic molded part according to claim 10 comprising the following additional step
a₂) layered application of the gel or dispersion to a surface, preferably by spraying and/or a 3D printing process, particularly preferably by binder jetting, material jetting, poly-jet or multi-jet modeling.

12. **Ceramic molded part** obtainable by a process as defined in claims 10 or 11.

13. **Use** of a combination of a silicate ceramic material selected from the group consisting of feldspar and leucite ceramics and a binder comprising a gelling agent with a crosslinked polymer in a gel or a dispersion, for the production of a ceramic molded part.

## Revendications

1. Kit de produits chimiques, comprenant les composants suivants :
A) un matériau céramique, comprenant un matériau céramique à base de silicate ou constitué de celui-ci,
B) un liant, comprenant un gélifiant ou constitué de celui-ci, le gélifiant étant un polymère réticulé,
**caractérisé en ce que**
le matériau céramique à base de silicate est sélectionné dans le groupe constitué de céramiques à base de feldspath et de céramiques à base de leucite.

2. Kit de produits chimiques selon la revendication 1, dans lequel le polymère du gélifiant comprend un ou plusieurs motifs de répétition, dans lequel de préférence au moins un parmi le ou les motifs de répétition présente un groupe fonctionnel chélatant.

3. Kit de produits chimiques selon la revendication 1 ou 2, dans lequel le polymère est un polymère superabsorbant, de préférence un copolymère comportant un motif de répétition acide (méth)acrylique et/ou (méth)acrylate de sodium.

4. Kit de produits chimiques selon l'une des revendications précédentes, dans lequel le kit contient le composant supplémentaire suivant :
C) un solvant et/ou un dispersant, qui est de préférence sélectionné dans le groupe constitué de l'eau et d'alcools.

5. Kit de produits chimiques selon l'une quelconque des revendications précédentes, dans lequel le kit contient le composant supplémentaire suivant :
D) une base, de préférence sélectionnée dans le groupe constitué d'hydroxydes de métaux alcalins et alcalino-terreux et de carbonates de métaux alcalins et alcalino-terreux.

6. Kit de produits chimiques selon l'une des revendications précédentes, dans lequel l'ensemble contient le composant supplémentaire suivant :
E) un conservateur, de préférence biocide.

7. Kit de produits chimiques selon l'une des revendications précédentes, dans lequel le kit contient le composant supplémentaire suivant :
F) un composant colorant, qui est de préférence sélectionné parmi Er₂O₃, Fe₂O₃, Co₃O₄, MnO₂, NiO₂, Cr₂O₃, Pr₂O₃, Tb₂O₃, Bi₂O₃ et des mélanges de ceux-ci.

8. Kit de produits chimiques selon l'une des revendications précédentes, dans lequel la proportion en poids du matériau céramique par rapport au poids du kit de produits chimiques se situe dans la plage comprise entre 50 et 90 % en poids, de préférence entre 60 et 80 % en poids.

9. Gel ou dispersion, comprenant le kit de produits chimiques tel que défini dans l'une des revendications précédentes.

10. Procédé de fabrication d'une pièce moulée en céramique, comprenant les étapes suivantes consistant à :
a₁) mélanger les composants d'un kit de produits chimiques tel que défini dans l'une des revendications précédentes afin d'obtenir un gel ou une dispersion,
b) de manière facultative : traiter thermiquement le gel ou la dispersion dans une plage de températures comprise entre 50 °C et 200 °C afin d'obtenir une ébauche de compact,
c) de manière facultative : éliminer le liant par traitement thermique de l'ébauche de compact dans une plage de températures comprise entre 250 °C et 350 °C afin d'obtenir une pièce brute,
d) fritter par traitement thermique dans une plage de températures comprise entre 700 °C et 1000 °C afin d'obtenir la pièce moulée en céramique.

11. Procédé de fabrication d'une pièce moulée en céramique selon la revendication 10, comprenant l'étape supplémentaire suivante consistant à :
a₂) appliquer par couches le gel ou la dispersion sur une surface, de préférence par une technique de pulvérisation et/ou par un procédé d'impression 3D, de manière particulièrement préférée par jet de liant, jet de matière, modelage PolyJet ou à jets multiples.

12. Pièce moulée en céramique pouvant être obtenue par un procédé tel que défini dans la revendication 10 ou 11.

13. Utilisation d'une combinaison d'un matériau céramique à base de silicate, sélectionné dans le groupe constitué de céramiques à base de feldspath et de céramiques à base de leucite, et d'un liant comprenant un gélifiant avec polymère réticulé dans un gel ou une dispersion afin de fabriquer une facette céramique.
